# EUROPEAN PATENT SPECIFICATION

(11) **EP 1 875 873 B1**
(45) Date of publication and mention of the grant of the patent: **05.05.2010**
(21) Application number: 07252540.5
(22) Date of filing: 22.06.2007
(51) Int. Cl.: A61B 17/32, A61B 17/34, B21D 28/00, B26B 9/00, B26B 21/00, A61B 17/00

(54) **Thin bladed obturator**
Obturator mit dünner Klinge
Obturateur à fine lame

(30) Priority: 07.07.2006 US 819245 P
(43) Date of publication of application: 09.01.2008
(60) Divisional application: 09001905.0
(73) Proprietor: Tyco Healthcare Group LP, Norwalk, Connecticut 06856 (US)
(72) Inventor: Wenchell, Thomas, Durham, CT 06422 (US)
(74) Representative: Alcock, David

(56) References cited:
- EP-A- 1 634 535
- EP-A1- 1 493 394
- WO-A-2004/089582
- US-A- 5 364 372
- US-A- 5 609 604

## Description

### BACKGROUND

### 2. Background of the Related Art

Minimally invasive procedures are continually increasing in number and variation. Forming a relatively small diameter temporary pathway to the surgical site is a key feature of most minimally invasive surgical procedures. The most common method of providing such pathway is by inserting a trocar assembly through the skin. In many procedures, the trocar assembly is inserted into an insufflated body cavity of a patient. In such procedures, the trocar assemblies with seal mechanisms are utilized to provide the necessary pathway to the surgical site while minimizing leakage of insufflation gases.

Trocar assemblies typically include an obturator which is removably inserted through a cannula. The obturator may incorporate a penetrating end defining a general pyramidal or frusto-conical shape and having a sharpened or blunt point. In the alternative, the obturator may incorporate a thin bladed member. Examples of obturator blades having a full width cutting edge are disclosed in the closest prior art documentU.S. Patent Nos. 5,364,372, assigned to Danks. Advantages of these thin bladed members include reduced penetration forces and-smaller openings in the incision thereby reducing patient trauma, recovery time, etc.

### SUMMARY

In accordance with one embodiment of the present disclosure, a process for manufacturing an obturator blade for a surgical obturator as defined in claim 1 includes the steps of providing an obturator blade blank, pressing the blade blank to form intersecting surfaces adjacent one side, preferably, both sides, of the blade blank and forming a cutting edge adjacent an area of intersection of the intersecting surfaces such that the peripheral coating edges terminate before the maximum width of the blade to thereby form an obturator blade for incorporation in a surgical obturator. The step of pressing preferably includes the step of coining the blade blank with at least one coining die to, e.g., form at least one arcuate surface adjacent the one side of the blade blank. The at least one arcuate surface is one of the intersecting surfaces. More preferably, the step of coining includes forming arcuate intersecting surfaces adjacent the one side of the blade blank. The arcuate intersecting surfaces may be substantially concave intersecting surfaces. Alternatively, the step of coining includes forming substantially planar intersecting surfaces adjacent the one side of the blade blank.

The step of forming may include the step of etching the blade blank adjacent the area of intersection of the intersecting surfaces. Preferably, the step of etching includes subjecting the blade blank to an acid bath.
In another embodiment, a process for manufacturing an obturator blade for a surgical obturator, includes the steps of providing an obturator blade blank, coining the blade blank to form first and second-pairs of substantially concave intersecting surfaces adjacent respective sides of the blade blank and forming cutting edges adjacent respective lines of intersection of the first and second pairs of the intersecting surfaces to thereby form an obturator blade for incorporation in a surgical obturator. The process may further include the step of arranging the first and second pairs of intersecting surfaces such that the lines of intersection taper inwardly relative to a longitudinal axis of the blade blank. The step of forming preferably includes the step of etching with, e.g., an acid bath, the blade blank adjacent the area of intersection of the intersecting surfaces.

In another embodiment, a surgical obturator blade is provided as defined in claims 17 and 18. The surgical obturator includes an obturator member having a distal end and a proximal end and an obturator blade member adjacent the distal end of the obturator-member. The blade member includes a peripheral cutting edge defined by first and second surfaces. The peripheral edge is relatively sharp adjacent a leading end of the blade-member and is relatively blunt toward a trailing edge of the blade member. In one aspect, the peripheral cutting edge-is dimensioned to transition from being relatively sharp adjacent the leading end to being relatively blunt toward the trailing end of the blade member. Preferably, the obturator blade member includes opposed peripheral cutting edges defined by two pairs of the first and second surfaces. Preferably, the first and second-surfaces are substantially concave. Alternatively, the first and second surfaces may be substantially planar. The obturator blade member includes a penetrating tip which may be pointed or blunt.

### BRIEF DESCRIPTION OF THE DRAWINGS

The foregoing features of the present disclosure will become more readily apparent and will be better understood by referring to the following detailed description of the preferred embodiments-which are described hereinbelow with reference to-the drawings wherein:
FIG. 1 is a perspective view of a trocar assembly in accordance with the present disclosure illustrating the cannula assembly and the obturator assembly;
FIG. 2 is a perspective view of the obturator assembly of the trocar assembly in accordance with the embodiment of FIG. 1;
FIG. 3 is a block diagram of a preferred process of manufacturing an obturator blade from obturator blade stock material in accordance with the embodiment of FIGS. 1-2;
FIG. 4 is a top plan view of the obturator blade blank subsequent to the stamping step of the preferred process in accordance with the embodiment of FIGS. 1-3;
FIG. 5 is a perspective view of a pair of lower coining dies utilized in the coining operation in accordance with the embodiment of FIGS. 1-3;
FIG. 6 is a top plan view of the lower pair of coining dies in accordance with the embodiment of FIGS. 1-4;
FIG. 7 is a top plan view of the obturator blade stock subsequent to the coining operation in accordance with the embodiment of FIGS. 1-6;
FIG. 8 is a top plan view of the obturator blade manufactured in accordance with the embodiment of FIGS. 1-5;
FIG. 9 is a side elevation view of the obturator blade manufactured in accordance with the embodiment of FIGS. 1-8;
FIG. 10 is a cross-sectional view of the obturator blade taken along section line 10-10 of FIG. 8 in accordance with the embodiment of FIGS. 1-9;
FIG. 11 is a cross-sectional view of the obturator blade taken-along section line 11-11 of FIG. 8 in accordance-with the embodiment of FIGS. 1-8;
FIG. 12 is a perspective view of another pair-of lower coining dies utilized in the coining operation in accordance with an alternate embodiment of the present disclosure;
FIG. 13 is a top plan view of the obturator blade manufactured in accordance with the embodiment of FIG. 12; and
FIG. 14 is a cross-sectional view of the obturator blade taken along section line 14-14 of FIG. 13 in accordance with the embodiment of FIGS. 12-13.

### DETAILED DESCRIPTION OF PREFERRED EMBODIMENTS

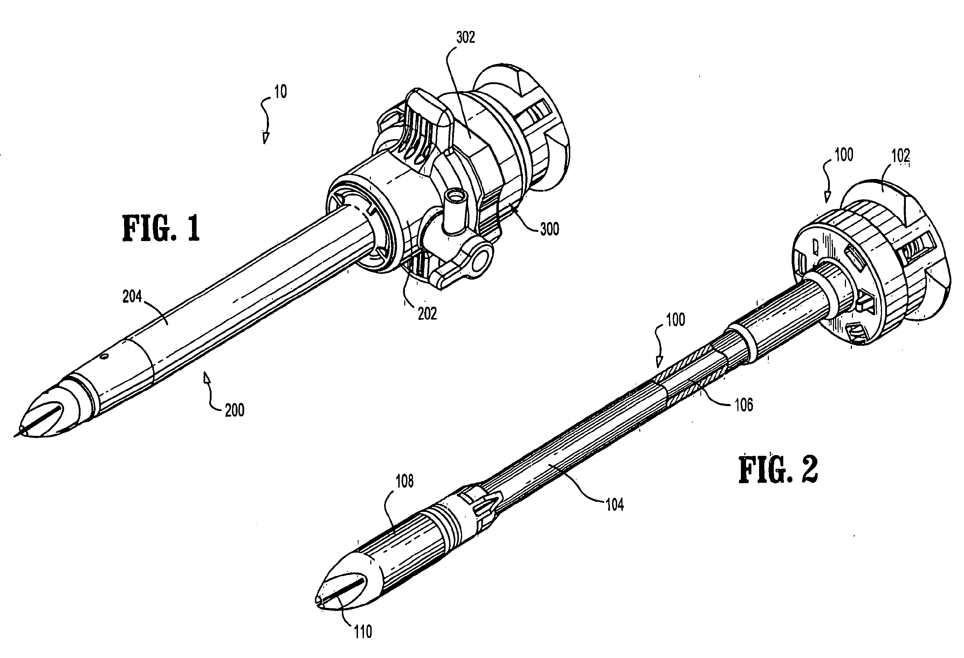

Referring now in detail to the drawing figures, in which like references numerals identify similar or identical elements, there is illustrated, in FIG. 1, a trocar assembly constructed -in accordance with a preferred embodiment of the-present disclosure and designated generally by reference numeral 10. Trocar assembly 10 is particularly adapted for-use- in minimally invasive surgical procedures such as endoscopic_or laparoscopic procedures. Generally, trocar assembly 10 includes two principal subassemblies, namely, obturator assembly 100 and cannula assembly 200.

Cannula assembly 200 may be any cannula assembly suitable for use in a laparoscopic surgical procedure. In one preferred embodiment, cannula assembly 200 includes cannula housing 202 and cannula sleeve 204 extending from the cannula housing 202. Either or both cannula housing 202 and cannula sleeve 204 may be transparent in part or in whole and are fabricated from biocompatible metal or polymeric material. Cannula assembly 200 may include an internal seal such as a duck-bill valve or other zero closure valves adapted to close in the absence of a surgical instrument to prevent passage of insufflation gases through the cannula assembly 200.

Trocar assembly 10 may also include a seal assembly 300 which is preferably releasably mounted to cannula housing 202. Means for releasably connecting seal assembly 300 to cannula housing 202 may include a bayonet coupling, threaded connection, latch, friction fit, tongue and groove arrangements, snap-fit, etc. Seal assembly 300 may include seal housing 302 and at least one internal seal which is adapted to form a fluid tight seal about an instrument inserted through the seal-assembly 2000. One suitable seal may be the fabric seal disclosed in commonly assigned U.S. Patent No. 6,702,787 to Racenet. The seal disclosed in the '787 patent may be a flat septum seal having a first layer of resilient material and a second fabric layer juxtaposed relative to the first layer. Further details of the seal may be ascertained by reference to the '787 patent. Seal assembly 300 may or may not be a component of cannula assembly 200. For example, the seal assembly may be a separate, removable assembly. In the alternative, the seal assembly may comprise an integral part of the cannula assembly 200-and not be removable.

Referring now to FIG. 2, in conjunction with FIG. 1, obturator assembly 100 includes obturator housing 102 and elongated obturator portion 104 extending distally from the obturator housing 102. Obturator housing 102 may include several housing components assembled together to define a unit dimensioned for grasping by the surgeon. Obturator portion 104 may incorporate internal obturator shaft 106 (shown in the cut-away of FIG. 2), sheath 108 coaxially mounted about the obturator shaft 106 and obturator blade 110 mounted to the distal end of the obturator shaft 106. Sheath 108 may be adapted for reciprocal longitudinal-movement relative to the obturator shaft 106 from a first extended position enclosing obturator blade 110 as shown in FIG. 1 to a second retracted position (not shown) to expose the obturator blade 110. Alternatively, obturator shaft 106 may be advanced in a longitudinal direction to expose obturator blade 110. Various latch mechanism or provisions may be incorporated into-obturator assembly 100 to prevent "arming" of obturator blade 110 until such time that the obturator assembly 100 is ready for use. Further details of obturator assembly with a latch mechanism suitable for use with the present disclosure is disclosed in commonly assigned U.S. Patent No. 6,319,266 to Stellon et al..

Obturator blade 110 is a flat or planar blade having a penetrating end with opposed cutting edges contiguous with the penetrating end and extending therefrom in a proximal dimetion. Obturator blade 110 is preferably manufactured in accordance with the methodology discussed hereinbelow.

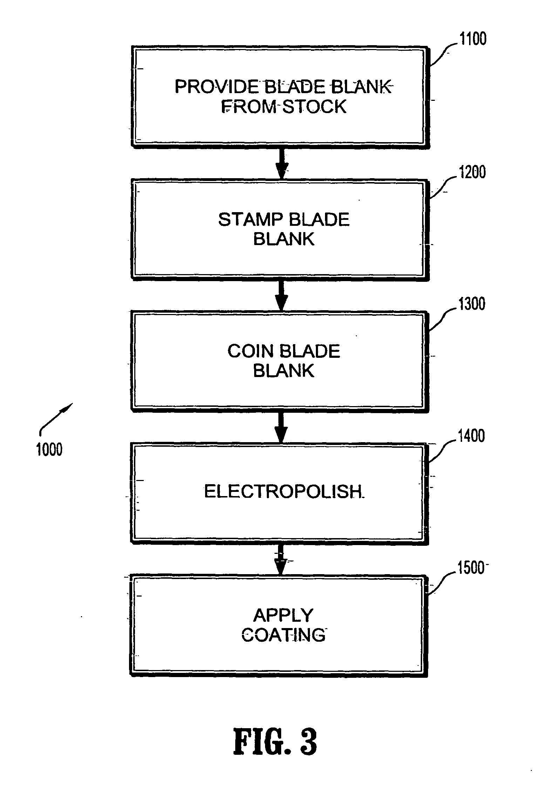

Referring now to the block diagram of FIG. 3, there is illustrated a preferred process 1000 for manufacture of obturator blade 110 for incorporation into obturator assembly 100. These steps are inclusive of 1) provide blade blank from stock (STEP 1100), 2) stamp blade blank (STEP 1200), 3) coin blade blank (STEP 1300), 4) electropolish blade (STEP 1400) and 5) apply coating (STEP 1500). The first step or operation (STEP 1100) in the process is to provide the blade blank. A blank in the form of, e.g., a flat or planar stock material having a predetermined length is provided. The blank is to be eventually formed into obturator blade 110. The blank may be cut from suitable stock, including stainless steel, titanium-or alloys of titanium. Preferably, the blank has the same thickness as the intended thickness of the finished obturator blade 110.

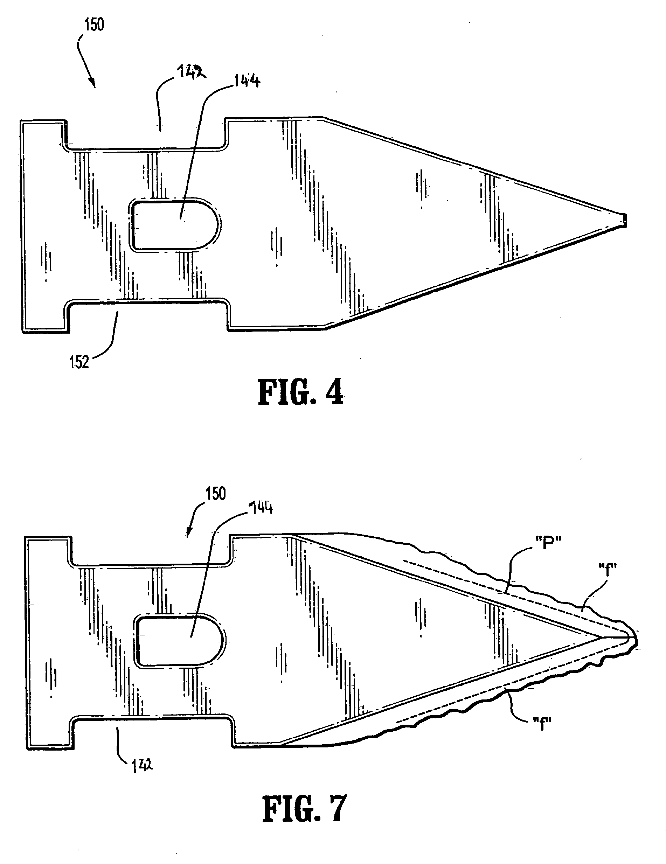

The second operation (STEP 1200) in the process is stamping the blade blank to define various apertures or recesses which facilitate mounting of the finished obturator blade 110 within obturator assembly 100 and also to form the penetrating end of the blade blank into its general triangular configuration. Any conventional stamping press and/or dies may be utilized. FIG. 4 illustrates in plan view, obturator blade blank 150 subsequent to the stamping step (STEP 1200). As illustrated, recesses 142 are formed in opposed side edges of blade blank 150. A central aperture 144 is formed in the interior area of blade blank 150. Recesses 142 and aperture 144 serve to mount obturator blade 110 to obturator shaft 106. Other configurations are envisioned as well. In addition, the stamping process (STEP 1200) provides the generally triangular configuration to the leading-end- of the blade blank 150.

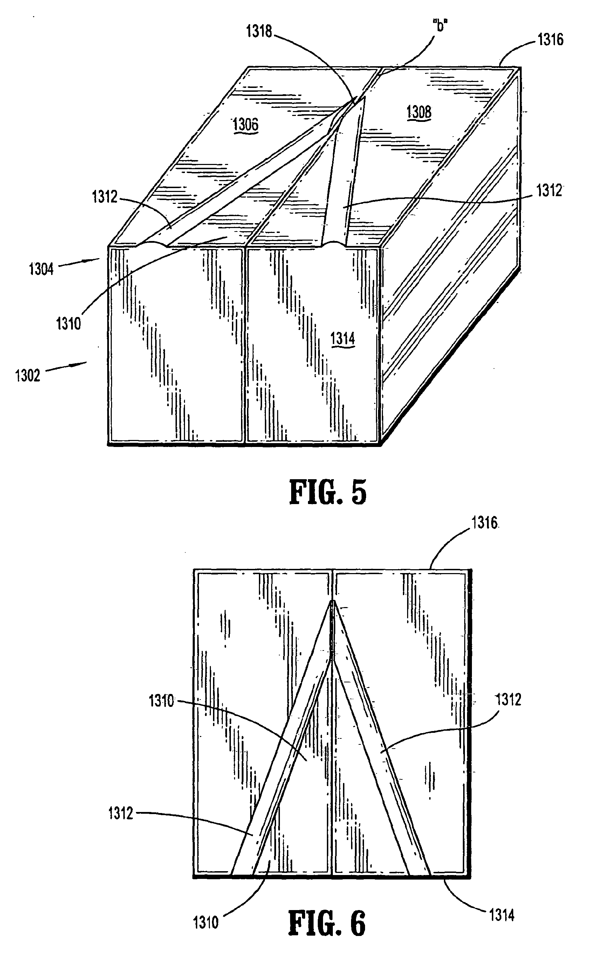

Once the blade blank is stamped, the next step (STEP 1300) in the process is to press the obturator blade blank 150 to generally form the cutting edges on the obturator blade blank 150. With reference now to FIGS. 5-6, the preferred pressing operation incorporates a coining die press 1302. Any conventional coining die press may be modified for use with the process of the present disclosure such as the apparatus disclosed in commonly assigned U.S. Patent No. 5,640,874 to Vecsey. The coining die press 1302 includes first and second pairs of dies, e.g., upper and lower pairs, constructed to form a cavity for the pressing operation. For illustrative purposes, only the lower pair 1304 of dies is shown in FIGS. 5-6. Lower pair 1304 of dies includes left and right dies 1306, 1308 which are placed in juxtaposed side by-side relation and held in such position by a fixture, clamp or the like associated with the coining press. Lower pair 1304 of dies 1306, 1308 defines a longitudinal bisecting axis "b" along the line of intersection of the left and right dies 1306, 1308 and, further defines an internal die surface which contacts the obturator blank 150 upon actuation of the coining press. The internal die surface of lower pair 1304 of dies 1306, 1308 includes a central substantially triangular planar surface 1310 and opposed coining surfaces 1312 on each side of the planar surface 1310. Each coining surface 1312 is substantially a raised arcuate, preferably, a convex coining surface and extends inwardly toward the bisecting axis "b" from a front surface 1314 of the pair 1304 of dies 1306, 1308 to a rear surface 1316 of the dies 1306,1308. When the left and right dies 1306, 1308 are assembled within the die chuck or fixture, coining surfaces 1312 define a general triangular shape generally corresponding to the triangular shape of obturator blade blank 150. A slight gap 1318 may exists at the apex of the triangle along the line of intersection of the-left and right dies 1306, 1308 to permit flash and material flow during the-coining process. This eventually becomes the location of an upper or lower cutting edge of the finished obturator blade 110. The upper pair of dies 1306, 1308 would be identical to lower pair 1302, with coining surfaces 1312 facing the lower pair 1302 when assembled in the coining press. Alternatively, the upper dies may be replaced with a flat punch which would produce a generally flat surface on the opposed surface of the blade blank 150. It is also envisioned that a single die unit may constitute the lower and upper dies. The dies are preferably formed of a carbide material although other materials are envisioned as well.
In operation, the obturator blade blank 150 is placed between the upper and lower pairs 1302 of dies 1304, 1306 and positioned such that the periphery of the triangular leading end of blade blank 150 is aligned with respective-coining surfaces 1312. The press is operated such that the upper and/or lower pairs of dies 1304, 1306 advance to engage the blade blank 150 thereby-causing coining surfaces 1312 to engage, swage- or coin the blade blank 150 along its peripheral-edges to generally form the cutting edges of the blade blank. FIG. 7 illustrates the appearance of blade blank 150 subsequent to the coining step (STEP 1300). As shown, the-coining process creates an overflow flash "f" on each side of the blade blank 150 of a defined thickness. The flash "f" results from blade stock material overflow at a location adjacent the coining triangular recessed area. The flash "f" extends radially outwardly from the normal perimeter (identified as "p") of the blade blank.

With reference again to the process depicted in FIG. 3, the next step in the process is an electro polish or etching operation (STEP 1400). The electro polish operation incorporates the-step of submerging the-obturator blade blank in an acid bath to acid etch the blade blank 150 to form, -finish and sharpen the cutting edges of the blade blank 150. The acid bath has a high amperage current of, e.g., 5-6 amps-introduced into the bath for a predetermined period of time. The high energy phase aggressively moves excess flash material from the blade blank 150 and sharpens the cutting edges. A second phase may be employed in this process is a low energy step and includes directing a relatively low amperage current of approximately 1 amp into the acid bath for about five minutes. This phase produces a matte-like finish on the blade blank 150. The matte finish facilitates retention of a subsequent coating which may be applied to the blade.

Subsequent to the etching operation 1400, blade blank 150 may then be coated with a suitable coating, e.g., a silicon coating, PTFE coating or Teflon to enhance passage through the tissue. Alternatively, blade blank 150 may be coated with a suitable antimicrobial coating.

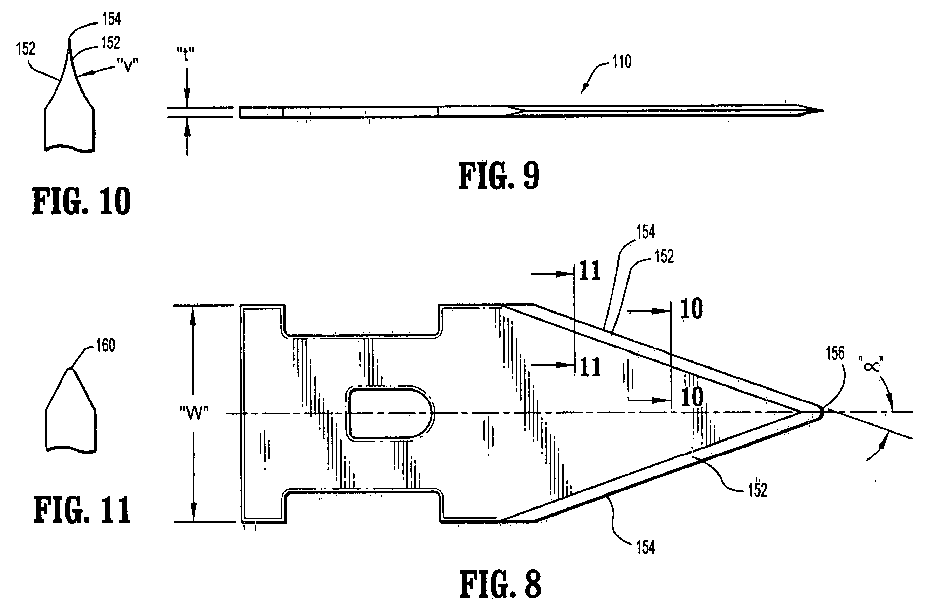

FIGS. 8-11 illustrate the-obturator blade 110 which is manufactured in accordance with the process 1000 described hereinabove. Obturator blade 110 is substantially thin defining a thickness "t" substantially less than a width "w". Obturator blade 110 includes pairs of opposed curved surfaces 152 formed into the blade 110 and defining peripheral-cutting edges 154 along the lines of intersection of the curved surfaces 152. Curved surfaces 152 are formed during the coining step (STEP 1300) of the process 100 and are generally concave in configuration corresponding in configuration to coining surfaces 1310 of dies 1306, 1308. Curved surfaces 152 preferably define a radius of curvature "v" ranging from about 05 inches to about 3.55 inches. Peripheral cutting edges 154 extend at an angle "α" relative to longitudinal axis. Angle "α" may range-from about 18° to about 22°, preferably, about 20°. Cutting edges 154 terminate adjacent penetrating point 156. Penetrating point 156 may be sharp or blunt. Extending rearward from penetrating point 156 are upper and lower cutting edges 158. Cutting edges 158 may be formed adjacent gap 1318 of dies 1306, 1308 (FIG.5) during the coining step (STEP 1300) and sharpened during the etching step (STEP 1400).

In accordance with the process 1000 of the present disclosure, cutting edges 154 do not extend completely across the maximum blade width "w". In particular, it is envisioned that the coining step (STEP 1300) can be modified to produce a blade whose periphery is generally blunt 160 or atraumatic approaching the trailing end of the triangular penetrating end of the blade 150. This configuration is best depicted in the cross-sectional view of FIG. 11 and may be of benefit in certain surgical procedures where expansion of the incision to its maximum diameter necessitates a non piercing approach. The transition from the sharp edge 154 to blunt edge 160 may be gradual or immediate. Curved surfaces 152 and cutting edges 152 of obturator blade 110 provide substantial benefits in penetrating and passing through tissue particularly -when compared-to obturator blades with flat surfaces.- Specifically, the concave configuration provides sharp edges and-a narrow profile, thereby resulting in reduced penetration and drag through-tissue.

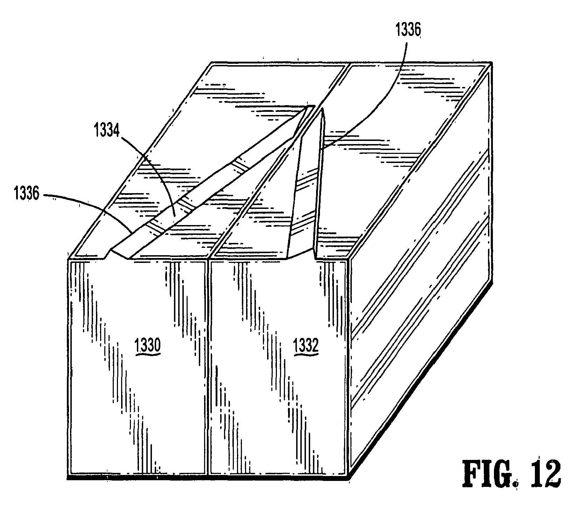

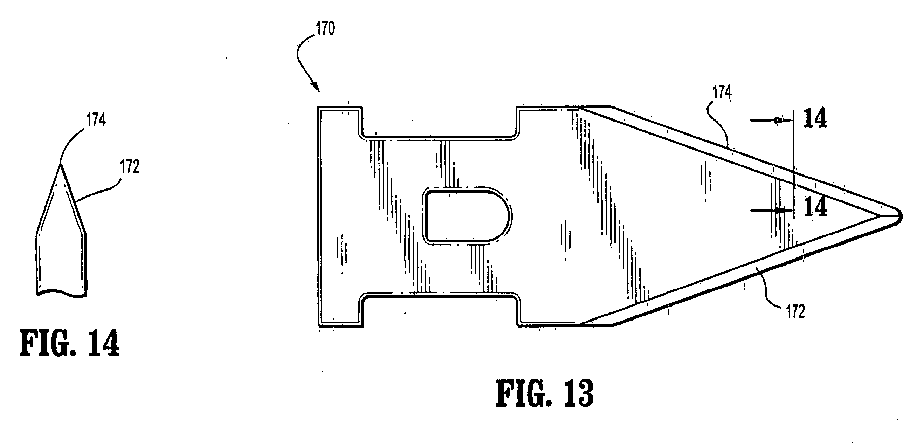

FIG. 12 illustrates an alternate die arrangement for use during the coining step. (STEP 1300). In accordance with this embodiment, coining dies 1330, 1302 incorporate coining surfaces 1334 which are generally triangular in cross-section defining substantially planar coining surfaces 1336. This arrangement will produce an obturator blade 170 with straight peripheral surfaces 172 which intersect to defining peripheral cutting edges 174 as shown in FIGS. 13-14. It is envisioned with this embodiment that the peripheral cutting edges terminate before the maximum width of the obturator blade to thereby provide blunt surfaces rearward of the cutting edges in the manner discussed hereinabove in connection with the embodiment of FIGS. 1-11.

It will be understood that various modifications and changes in form and detail may be made to the embodiments of the present disclosure without departing from the spirit and scope of the invention. Therefore, the above description should not be construed as limiting the invention but merely as exemplifications of preferred embodiments thereof. Those skilled in the art will envision other modifications within the scope of the present invention as defined by the claims appended hereto. Having thus described the invention with the details and particularity required by the patent laws, what is claimed and desired protected is set forth in the appended claims.

## Claims

1. A process for manufacturing an obturator blade (110) having opposed cutting edges contiguous with a penetrating end for a surgical obturator (10), comprising the steps of:
providing an obturator blade blank (150);
pressing the blade blank (150) to form intersecting surfaces (152, 172) adjacent one side of the blade blank (150); and
forming peripheral cutting edges (154) adjacent an area of intersection of the intersecting surfaces **characterised in that** the peripheral cutting edges (154) are formed to terminate before the maximum width of the blade (110) to thereby form an obturator blade (110) for incorporation in a surgical obturator (10).

2. The process according to claim 1 wherein the step of pressing includes the step of coining the blade blank (150) with at least one coining die (1302).

3. The process according to claim 2 wherein the step of coining includes forming at least one arcuate surface (152) adjacent the one side of the blade blank (150), the at least one arcuate surface being one of the intersecting surfaces (152).

4. The process according to claim 3 wherein the step of coining includes forming arcuate intersecting surfaces (152) adjacent the one side of the blade blank (150).

5. The process according to claim 2 wherein the step of coining includes forming substantially concave intersecting surfaces (152) adjacent the one side of the blade blank (150).

6. The process according to claim 2 wherein the step of coining includes forming substantially planar intersecting surfaces (172) adjacent the one side of the blade blank (150).

7. The process according to claim 1 wherein the step of forming includes the step of etching the blade blank (150) adjacent the area of intersection of the intersecting surfaces.

8. The process according to claim 7 wherein the step of etching includes the step of subjecting the blade blank (150) to an acid bath.

9. The process according to claim 1 wherein the step of pressing includes forming intersecting surfaces (152, 172) adjacent the other side of the blade blank (150).

10. The process according to any one of claims 1 to 9 wherein the obturator blade (110) is subjected to electro-polishing or etching.

11. The process according to claim 10 wherein the obturator blade (110) is coated with a suitable coating material.

12. The process according to claim 11 wherein the suitable coating material is selected from the group consisting of silicon coating, PTFE coating, Teflon coating or an antimicrobial coating.

13. A process for manufacturing an obturator blade (110) for a surgical obturator (10) according to claim 1, comprising the steps of:
coining the blade blank (150) to form first and second pairs of substantially concave intersecting surfaces (152) adjacent respective-sides of the blade blank (150); and
forming peripheral cutting edges adjacent respective lines of intersection of the first and second pairs of the intersecting surfaces (154) such that the peripheral cutting edges terminate before the maximum width of the blade (110) to thereby form an obturator blade (110) for incorporation in a surgical obturator (10).

14. The process-according to claim 13 including the step of arranging the first and second pairs of intersecting surfaces (152) such that the lines of intersection taper inwardly relative to a longitudinal axis of the blade blank (150).

15. The process according to claim 14 wherein the step of forming includes the step of etching the blade blank (150) adjacent the area of intersection of the intersecting surfaces (152).

16. The process according to claim 15 wherein the step of etching includes the step of subjecting the blade blank (150) to an acid bath.

17. An obturator blade (110) having a peripheral cutting edge terminating before the maximum width of the blade manufactured according to the manufacturing process of claim 1,

18. An obturator blade (110) according to claim 17, wherein the blade is etched according to the process of claim 16.

## Patentansprüche

1. Prozess zur Herstellung einer Obturatorklinge (110) mit gegenüberliegenden Schneidkanten nahe eines eindringenden Endes für einen chirurgischen Obturator (10), der die Schritte aufweist:
Bereitstellen eines Obturatorklingenrohlings (150),
Pressen des Klingenrohlings (150), um sich schneidende Flächen (152, 172) neben einer Seite des Klingenrohlings (150) zu bilden, und
Bilden äußerer Schneidkanten (154) neben einem Bereich des Schneidens der sich schneidenden Flächen, **dadurch gekennzeichnet, dass** die äußeren Schneidkanten (154) derart ausgebildet sind, dass sie vor der maximalen Breite der Klinge (110) enden, um hierdurch eine Obturatorklinge (110) zu bilden für den Einsatz in einem chirurgischen Obturator (10).

2. Verfahren nach Anspruch 1, bei dem der Schritt des Pressens den Schritt des Prägens des Klingenrohlings (150) mit zumindest einem Prägestempel (1302) beinhaltet.

3. Verfahren nach Anspruch 2, bei dem der Schritt des Prägens das Ausbilden zumindest einer gekrümmten Fläche (152) neben der einen Seite des Klingenrohlings (150) beinhaltet, wobei die zumindest eine gekrümmte Fläche eine der sich schneidenden Flächen (152) ist.

4. Verfahren nach Anspruch 3, bei dem der Schritt des Prägens das Ausbilden gekrümmter sich schneidender Flächen (152) neben der einen Seite des Klingenrohlings (150) beinhaltet.

5. Verfahren nach Anspruch 2, bei dem der Schritt des Prägens das Ausbilden im Wesentlichen konkaver sich schneidender Flächen (152) neben der einen Seite des Klingenrohlings (150) beinhaltet.

6. Verfahren nach Anspruch 2, bei dem der Schritt des Prägens das Ausbilden im Wesentlichen ebener sich schneidender Oberflächen (172) neben der einen Seite des Klingenrohlings (150) beinhaltet.

7. Verfahren nach Anspruch 1, bei dem der Schritt des Ausbildens den Schritt des Ätzens des Klingenrohlings (150) neben dem Schnittbereich der sich schneidenden Oberflächen beinhaltet.

8. Verfahren nach Anspruch 7, bei dem der Schritt des Ätzens den Schritt des Aussetzens des Klingenrohlings (150) eines Säurebades beinhaltet.

9. Verfahren nach Anspruch 1, bei dem der Schritt des Pressens das Ausbilden sich schneidender Oberflächen (152, 172) neben der anderen Seite des Klingenrohlings (150) beinhaltet.

10. Verfahren nach einem der Ansprüche 1 bis 9, bei dem die Obturatorklinge (110) dem Elektropolieren oder Ätzen ausgesetzt ist.

11. Verfahren nach Anspruch 10, bei dem die Obturatorklinge (110) mit einem geeigneten Beschichtungsmaterial beschichtet ist.

12. Verfahren nach Anspruch 11, bei dem das geeignete Beschichtungsmaterial aus der Gruppe ausgewählt ist, die aus Siliziumbeschichtung, PTFE-Beschichtung, Teflonbeschichtung oder einer antimikrobiellen Beschichtung besteht.

13. Verfahren zur Herstellung einer Obturatorklinge (110) für einen chirurgischen Obturator (10) nach Anspruch 1, das die Schritte aufweist:
Prägen des Klingenrohlings (150), um erste und zweite Paare aus im Wesentlichen konkaven sich schneidenden Flächen (152) neben den entsprechenden Seiten des Klingenrohlings (150) zu bilden, und Ausbilden äußerer Schneidkanten neben den entsprechenden Schnittlinien des ersten und zweiten Paares der sich schneidenden Flächen (154), so dass die äußeren Schneidkanten vor der maximalen Breite der Klinge (110) enden, um hierdurch eine Obturatorklinge (110) für den Einsatz in einem chirurgischen Obturator (10) zu bilden.

14. Verfahren nach Anspruch 13, einschließlich des Schrittes der Anordnung des ersten und zweiten Paares der sich schneidenden Oberflächen (152), so dass die Schnittlinien sich nach innen relativ zu einer Längsachse des Klingenrohlings (150) verjüngen.

15. Verfahren nach Anspruch 14, bei dem der Schritt des Formens den Schritt des Ätzens des Klingenrohlings (150) neben dem Bereich des Schnittes der sich schneidenden Oberflächen (152) aufweist.

16. Verfahren nach Anspruch 15, bei dem der Schritt des Ätzens den Schritt des Aussetzens des Klingenrohlings (150) eines Säurebades beeinhaltet.

17. Obturatorklinge (110) mit einer äußeren Schneidkante, die vor der maximalen Breite der Klinge endet, hergestellt nach dem Herstellungsverfahren nach Anspruch 1.

18. Obturatorklinge (110) nach Anspruch 17, bei der die Klinge entsprechend dem Verfahren nach Anspruch 16 geätzt ist.

## Revendications

1. Procédé de fabrication d'une lame (110) d'obturateur, destinée à un obturateur chirurgical (10) et munie d'arêtes de coupe opposées, contiguës à une extrémité de pénétration, comprenant les étapes consistant à :
fournir une ébauche (150) de lame d'obturateur ;
presser l'ébauche de lame (150), pour former des surfaces sécantes (152, 172) au voisinage direct de l'un des côtés de ladite ébauche de lame (150) ; et
former des arêtes périphériques de coupe (154) au voisinage direct d'une zone d'intersection des surfaces sécantes, **caractérisé par le fait que** lesdites arêtes périphériques de coupe (154) sont façonnées de manière à s'achever avant la largeur maximale de la lame (110), pour former ainsi une lame (110) d'obturateur dédiée à l'intégration dans un obturateur chirurgical (10).

2. Procédé selon la revendication 1, dans lequel l'étape de pressage inclut l'étape de matriçage de l'ébauche de lame (150) à l'aide d'au moins une matrice de frappe (1302).

3. Procédé selon la revendication 2, dans lequel l'étape de matriçage inclut le formage d'au moins une surface arquée (152) au voisinage direct de l'un, précité, des côtés de l'ébauche de lame (150), ladite surface arquée, prévue au minimum, étant l'une (152) des surfaces sécantes.

4. Procédé selon la revendication 3, dans lequel l'étape de matriçage inclut le formage de surfaces sécantes arquées (152) au voisinage direct de l'un, précité, des côtés de l'ébauche de lame (150).

5. Procédé selon la revendication 2, dans lequel l'étape de matriçage inclut le formage de surfaces sécantes (152), substantiellement concaves, au voisinage direct de l'un, précité, des côtés de l'ébauche de lame (150).

6. Procédé selon la revendication 2, dans lequel l'étape de matriçage inclut le formage de surfaces sécantes (172), substantiellement planes, au voisinage direct de l'un, précité, des côtés de l'ébauche de lame (150).

7. Procédé selon la revendication 1, dans lequel l'étape de formage inclut l'étape de traitement corrosif de l'ébauche de lame (150) au voisinage direct de la zone d'intersection des surfaces sécantes.

8. Procédé selon la revendication 7, dans lequel l'étape de traitement corrosif inclut l'étape d'exposition de l'ébauche de lame (150) aux effets d'un bain acide.

9. Procédé selon la revendication 1, dans lequel l'étape de pressage inclut le formage de surfaces sécantes (152, 172) au voisinage direct de l'autre côté de l'ébauche de lame (150).

10. Procédé selon l'une quelconque des revendications 1 à 9, dans lequel la lame (110) d'obturateur est soumise à un polissage électrolytique ou à un traitement corrosif.

11. Procédé selon la revendication 10, dans lequel la lame (110) d'obturateur est revêtue d'un matériau adéquat de revêtement.

12. Procédé selon la revendication 11, dans lequel le matériau adéquat de revêtement est sélectionné au sein du groupe comprenant un revêtement de silicone, un revêtement de PTFE, un revêtement de Téflon ou un revêtement anti-microbien.

13. Procédé de fabrication d'une lame (110) d'obturateur destinée à un obturateur chirurgical (10), conformément à la revendication 1, comprenant les étapes consistant à :
matricer l'ébauche de lame (150) pour former des premières et secondes paires de surfaces sécantes (152) substantiellement concaves, au voisinage direct de côtés respectifs de ladite ébauche de lame (150) ; et
façonner des arêtes périphériques de coupe au voisinage direct de lignes respectives d'intersection des premières et secondes paires desdites surfaces sécantes (152), de telle sorte que lesdites arêtes périphériques de coupe s'achèvent avant la largeur maximale de la lame (110), pour former ainsi une lame (110) d'obturateur dédiée à l'intégration dans un obturateur chirurgical (10).

14. Procédé selon la revendication 13, englobant l'étape consistant à agencer les premières et secondes paires de surfaces sécantes (152) de façon telle que les lignes d'intersection fassent cône, vers l'intérieur, vis-à-vis d'un axe longitudinal de l'ébauche de lame (150).

15. Procédé selon la revendication 14, dans lequel l'étape de formage inclut l'étape de traitement corrosif de l'ébauche de lame (150) au voisinage direct de la zone d'intersection des surfaces sécantes (152).

16. Procédé selon la revendication 15, dans lequel l'étape de traitement corrosif inclut l'étape d'exposition de l'ébauche de lame (150) aux effets d'un bain acide.

17. Lame (110) d'obturateur, munie d'une arête périphérique de coupe s'achevant avant la largeur maximale de la lame fabriquée conformément au procédé de fabrication selon la revendication 1.

18. Lame (110) d'obturateur, selon la revendication 17, ladite lame étant corrodée conformément au procédé selon la revendication 16.
